# EUROPEAN PATENT SPECIFICATION

(11) **EP 4 449 142 B1**
(45) Date of publication and mention of the grant of the patent: **23.09.2026**
(21) Application number: 22748440.9
(22) Date of filing: 13.07.2022
(51) Int. Cl.: G01R 31/3835, G01R 31/392

(54) **BATTERY STATE OF HEALTH CHECK**
BATTERIEZUSTANDSÜBERPRÜFUNG
CONTRÔLE DE L'ÉTAT DE SANTÉ D'UNE BATTERIE

(30) Priority: 16.12.2021 US 202117553351
(43) Date of publication of application: 23.10.2024
(73) Proprietor: Medtronic, Inc., Minneapolis, Minnesota 55432 (US)
(72) Inventor: CHEN, Kaimin, Minneapolis, Minnesota 55432 (US); DONAKOWSKI, Martin D., Minneapolis, Minnesota 55432 (US)
(74) Representative: Zimmermann & Partner Patentanwälte mbB
(86) International application number: PCT/IB2022/056465
(87) International publication number: WO 2023/111696

(56) References cited:
- US-A1- 2021 234 386
- US-B2- 10 416 238

## Description

### FIELD

The present disclosure relates to, among other things, batteries or electrochemical cells.

### TECHNICAL BACKGROUND

Batteries may include one or more electrochemical cells. Each electrochemical cell includes an anode (e.g., a negative electrode), a cathode (e.g., a positive electrode), and an electrolyte provided within a case or housing. A separator made from a porous polymer or other suitable material may also be provided intermediate or between the anode and the cathode to prevent direct contact between the anode and the cathode. The anode includes a current collector having an active material provided thereon, and the cathode includes a current collector having an active material provided thereon. The current collector may be the battery case or housing.

Zinc/silver oxide batteries may have a relatively flat discharge voltage shape. In other words, the voltage of zinc/silver oxide batteries may vary by less than 20 millivolts (e.g., between about 1.57 volts to about 1.55 volts) until the capacity of such batteries is discharged to about 20 percent of capacity (e.g., 20 percent state of charge). At about 20 percent capacity, the voltage of zinc/silver oxide may begin to drop precipitously. Additionally, the voltage of zinc/silver oxide batteries may both decrease and increase during the first 80 percent of capacity discharge. This may result in zinc/silver oxide batteries having the same or similar voltage when such batteries are at a state of charge over 80 percent when compared to such batteries at a state of charge between 50 percent to 20 percent. Accordingly, it may be difficult to determine a remaining capacity or a state of health of zinc/silver oxide batteries based on a present voltage level of such batteries alone.
US 2021/234386 A1 relates to a battery management system and method.

### BRIEF SUMMARY

According to the present invention, accurate determination of the health of a battery is determined based on two voltage measurements of the battery and a rate of voltage change of the battery. A first voltage of the battery is measured and compared to a voltage threshold. If the first voltage is less than the voltage threshold, the battery may be at a capacity less than 20 percent and deemed unsuitable for use. If the first voltage is greater than the voltage threshold, additional steps are taken to determine a state of health of the battery. The battery is allowed to discharge for a predetermined time period beginning when the first voltage was measured. A second voltage is measured at the conclusion of the predetermined time period. The first voltage, the second voltage, and the predetermined time period are used to determine a rate of voltage change of the battery during the predetermined time period. The rate of voltage change is used to estimate a remaining capacity of the battery and/or a state of health of the battery. Whether the battery is suitable for use may be determined based on the remaining capacity of the battery and/or the state of health of the battery.

Described herein, among other things, are a method and system for determining the health of batteries that have relatively flat voltage discharge curves such as, for example, zinc/silver oxide batteries. Measuring a first voltage of the battery and comparing the first voltage to a threshold can quickly identify batteries that are unsuitable for use (e.g., at less than 20 percent remaining capacity) without additional testing. Determining a rate of voltage change for batteries with a first voltage over a voltage threshold allows an accurate estimation of the remaining capacity and/or state of health of the battery. Additionally, determining that the first voltage is greater than the voltage threshold before determining the rate of voltage change can prevent healthy batteries from being deemed unsuitable.

In an example useful for understanding the invention, the present disclosure describes a method comprising measuring a first voltage of a battery and determining the first voltage is equal to or greater than a voltage threshold level. The method further includes measuring a second voltage of the battery at a conclusion of a predetermined time period in response to the first voltage being equal to or greater than the voltage threshold level, the predetermined time period beginning when the first voltage is measured. The method further includes determining a rate of voltage change based on the first voltage, the second voltage, and the predetermined time period. The method further includes determining a state of health of the battery based on the rate of voltage change.

The present invention provides a battery measurement system comprising a positive node, a negative node, a voltage sensor, and a computing apparatus. The positive node is configured to operatively couple to a positive terminal of a battery and provide a first voltage signal indicative of a positive terminal voltage of the battery. The negative node is configured to operatively couple to a negative terminal of the battery and provide second voltage signal indicative of a negative terminal voltage of the battery. The voltage sensor is operatively coupled to the positive node and the negative node to receive the first and second voltage signal. The voltage sensor is configured to determine a voltage of the battery based on the first and second voltage signals and provide a battery voltage signal. The computing apparatus comprises one or more processors and is operatively coupled to the voltage sensor. The computing apparatus is configured to measure a first voltage of the battery and determine the initial measured voltage is equal to or greater than a voltage threshold level. The computing apparatus is further configured to measure a second voltage of the battery at a conclusion of a predetermined time period in response to the initial measured voltage being equal to or greater than the voltage threshold level, the predetermined time period beginning when the first voltage is measured. The computing apparatus is further configured to determine a rate of voltage change based on the first voltage, the second voltage, and the predetermined time period. The computing apparatus is further configured to determine a state of health of the battery based on the rate of voltage change.

Advantages and additional features of the subject matter of the present disclosure will be set forth in the detailed description which follows, and in part will be readily apparent to those skilled in the art from that description or recognized by practicing the subject matter of the present disclosure as described herein, including the detailed description which follows, the claims, as well as the appended drawings.

It is to be understood that both the foregoing general description and the following detailed description present embodiments of the subject matter of the present disclosure, and are intended to provide an overview or framework for understanding the nature and character of the subject matter of the present disclosure as it is claimed. The accompanying drawings are included to provide a further understanding of the subject matter of the present disclosure and are incorporated into and constitute a part of this specification. The drawings illustrate various embodiments of the subject matter of the present disclosure and together with the description serve to explain the principles and operations of the subject matter of the present disclosure. Additionally, the drawings and descriptions are meant to be merely illustrative and are not intended to limit the scope of the claims in any manner.

### BRIEF DESCRIPTION OF THE DRAWINGS

The following detailed description of specific embodiments of the present disclosure can be best understood when read in conjunction with the following drawings, in which:
**FIG. 1** is a schematic block diagram of a battery measurement system;
**FIG. 2** is another schematic block diagram of the battery measurement system of **FIG. 1****;**
**FIG. 3** is a graph depicting a voltage of a zinc/silver oxide battery as such battery is discharged;
**FIG. 4** is another graph depicting a voltage of a zinc/silver oxide battery as such battery is discharged;
**FIG. 5** is a schematic flow diagram of a method or process for determining the health of a battery; and
**FIG. 6** is a top down view of an implantable medical device.

The schematic drawing is not necessarily to scale.

### DETAILED DESCRIPTION

Reference will now be made in greater detail to various embodiments of the subject matter of the present disclosure, some embodiments of which are illustrated in the accompanying drawings. Like numbers used in the figures refer to like components and steps. However, it will be understood that the use of a number to refer to a component in a given figure is not intended to limit the component in another figure labeled with the same number. In addition, the use of different numbers to refer to components in different figures is not intended to indicate that the different numbered components cannot be the same or similar to other numbered components.

The state of health of a battery with a relatively flat voltage curve can be determined based on a voltage of the battery and a rate of voltage change of the battery. According to the present invention, a first voltage of a battery is measured and compared to a voltage threshold level. If the first voltage is greater than or equal to the voltage threshold, the battery is allowed to discharge over a predetermined time period and a second voltage of the battery is measured at a conclusion of the predetermined time period. For example, the battery may be incorporated in a device that may draw some amount of power, thereby discharging the battery, even if the device is not fully activated or operational. Further, for example, the battery may be allowed to self-discharge. Such predetermined time period begins when the first voltage is measured. The rate of voltage change is determined based on the first voltage, the second voltage, and the predetermined time period. If the first voltage of the battery is less than the voltage threshold, the battery may be deemed unsuitable for use without conducting additional measurements.

As used herein, the term "relatively flat voltage curve" refers to batteries that have voltages that vary less than 20 millivolts above 20 percent state of charge and less than 98 percent of charge.

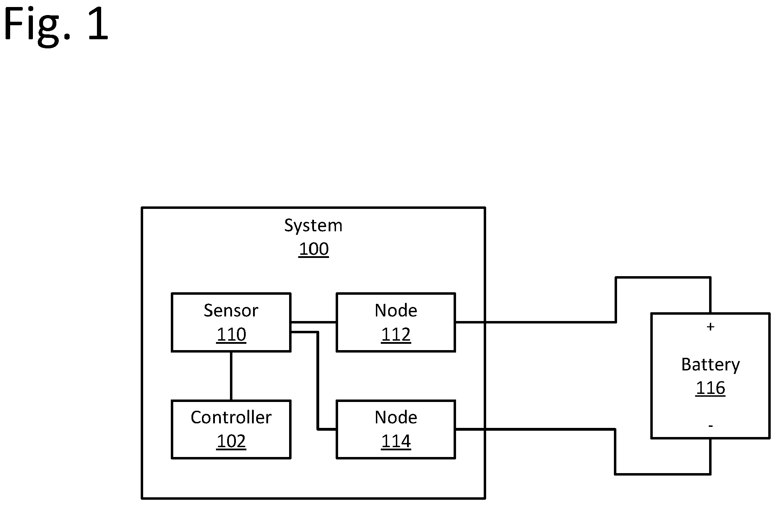

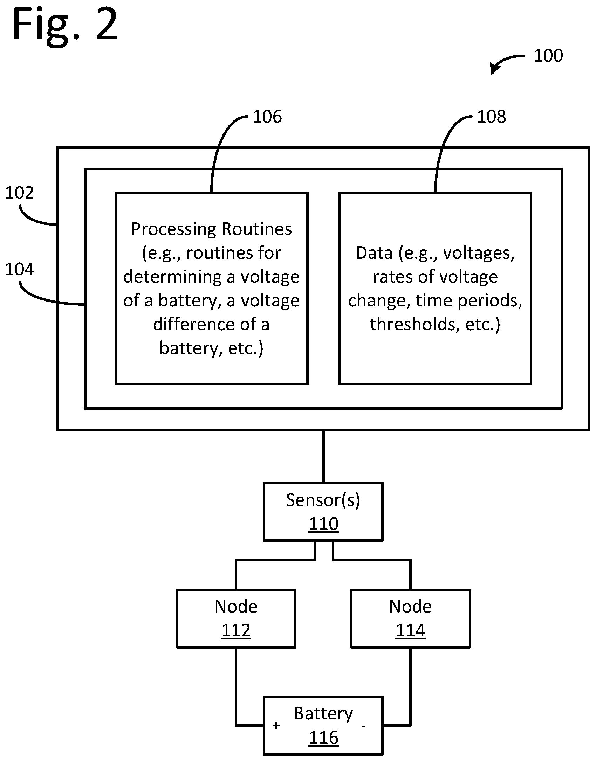

**FIG. 1** and 2 depict schematic block diagrams of a battery measurement system **100.** **FIG. 1** shows a schematic block diagram of the battery management system **100.** **FIG. 2** shows a schematic block diagram of the battery measurement system **100** that includes greater detail of a computing apparatus **102.**

The battery measurement system **100** is configured to determine a health of a battery (e.g., battery **116)** using methods or processes described herein. The battery measurement system **100** includes a computing apparatus **102,** a voltage sensor **110,** a positive node **112,** and a negative node **114.** The battery measurement system **100** may include the battery **116.** The battery **116** may include a positive terminal "+" and a negative terminal "-" corresponding to a cathode and anode of the battery **116** respectively.

According to the present invention, the positive node **112** is configured to operatively couple to the positive terminal of the battery **116** and provide a first voltage signal indicative of a voltage of the positive terminal. The negative node **114** is configured to operatively couple to a negative terminal of the battery and provide a second voltage signal indicative of the voltage of the negative terminal. The nodes **112, 114** may each include one or more contacts, leads, probes, conductors, clamps, or other apparatus to operatively couple to a battery terminal and provide a signal indicative of the voltage of such battery terminal. The nodes **112, 114** may include any suitable conductive material to allow the nodes **112, 114** to have substantially the same electrical potential (e.g., voltage) as an object (e.g., a battery terminal) that the nodes **112, 114** are in contact with or otherwise operatively coupled to.

The voltage sensor **110** is operatively coupled to the nodes **112, 114** to receive the first voltage signal and the second voltage signal. The voltage sensor **110** is configured to determine a voltage based on the first voltage signal and the second voltage signal. The voltage determined by the voltage sensor **110** may be the voltage difference between the first voltage signal and the second voltage signal. The voltage sensor **110** is configured to provide a battery voltage signal. The battery voltage signal may be indicative of the determined voltage.

The computing apparatus **102** is operatively coupled to the voltage sensor **110.** The computing apparatus includes one or more processors. The computing apparatus **102** is configured to determine a state of health of the battery **116** as described herein. The computing apparatus **102** is configured to measure a first voltage of the battery **116** using the voltage sensor **110** and determine the first voltage is equal to or greater than a voltage threshold level. The computing apparatus **102** is further configured to measure a second voltage of the battery **116** at a conclusion of a predetermined time period in response to the initial measured voltage being equal to or greater than the voltage threshold level. The predetermined time period begins when the first voltage is measured. The computing apparatus **102** is further configured to determine a rate of voltage change based on the first voltage, the second voltage, and the predetermined time period. The computing apparatus **102** is further configured to determine the state of health of the battery **116** based on the rate of voltage change.

Further, the computing apparatus **102** includes data storage **104.** Data storage **104** allows for access to processing programs or routines **106** and one or more other types of data **108** that may be employed to carry out the techniques, processes, and algorithms of determining a health of battery. For example, processing programs or routines **106** may include programs or routines for measuring a battery voltage, comparing a battery voltage to a threshold, determining a rate of voltage change, estimating a capacity of a battery determining a state of health of a battery, computational mathematics, matrix mathematics, Fourier transforms, compression algorithms, calibration algorithms, image construction algorithms, inversion algorithms, signal processing algorithms, normalizing algorithms, deconvolution algorithms, averaging algorithms, standardization algorithms, comparison algorithms, vector mathematics, or any other processing required to implement one or more embodiments as described herein.

Data **108** may include, for example, voltage data, voltage curve data, rate of voltage change data, time stamp data, predetermined time period data, temperature data, charging current data, state of health data, state of charge data, thresholds, arrays, meshes, grids, variables, counters, statistical estimations of accuracy of results, results from one or more processing programs or routines employed according to the disclosure herein (e.g., determining an age of a battery, determining a charging voltage of a battery, etc.), or any other data that may be necessary for carrying out the one or more processes or techniques described herein.

In one or more embodiments, the battery measurement system **100** may be controlled using one or more computer programs executed on programmable computers, such as computers that include, for example, processing capabilities (e.g., microcomputing apparatus, programmable logic devices, etc.), data storage (e.g., volatile or non-volatile memory and/or storage elements), input devices, and output devices. Program code and/or logic described herein may be applied to input data to perform functionality described herein and generate desired output information. The output information may be applied as input to one or more other devices and/or processes as described herein or as would be applied in a known fashion.

The programs used to implement the processes described herein may be provided using any programmable language, e.g., a high-level procedural and/or object orientated programming language that is suitable for communicating with a computer system. Any such programs may, for example, be stored on any suitable device, e.g., a storage media, readable by a general or special purpose program, computer or a processor apparatus for configuring and operating the computer when the suitable device is read for performing the procedures described herein. In other words, at least in one embodiment, the battery measurement system **100** may be controlled using a computer readable storage medium, configured with a computer program, where the storage medium so configured causes the computer to operate in a specific and predefined manner to perform functions described herein.

**The** computing apparatus **102** may be, for example, any fixed or mobile computer system (e.g., a personal computer or minicomputer). In at least one embodiment, the computing apparatus **102** is part of a device such as, for example, an implantable medical device. The exact configuration of the computing apparatus is not limiting and essentially any device capable of providing suitable computing capabilities and control capabilities (e.g., control the implementation of a state of health determination process of the battery measurement system **100,** the acquisition of data, such as voltage measurements or sensor data) may be used. Additionally, the computing apparatus **102** may be incorporated in a housing of the battery measurement system 100. Further, various peripheral devices, such as a computer display, mouse, keyboard, memory, printer, scanner, etc. are contemplated to be used in combination with the computing apparatus **102.** Further, in one or more embodiments, the data **108** (e.g., voltage data, voltage curve data, rate of voltage change data, time stamp data, predetermined time period data, temperature data, charging current data, state of health data, state of charge data, thresholds, arrays, meshes, grids, etc.) may be analyzed by a user, used by another machine that provides output based thereon, etc. As described herein, a digital file may be any medium (e.g., volatile or non-volatile memory, a CD-ROM, a punch card, magnetic recordable tape, etc.) containing digital bits (e.g., encoded in binary, trinary, etc.) that may be readable and/or writeable by computing apparatus **102** described herein. Also, as described herein, a file in user-readable format may be any representation of data (e.g., ASCII text, binary numbers, hexadecimal numbers, decimal numbers, audio, graphical) presentable on any medium (e.g., paper, a display, sound waves, etc.) readable and/or understandable by a user.

In view of the above, it will be readily apparent that the functionality as described in one or more embodiments according to the present disclosure may be implemented in any manner as would be known to one skilled in the art. As such, the computer language, the computer system, or any other software/hardware that is to be used to implement the processes described herein shall not be limiting on the scope of the systems, processes or programs (e.g., the functionality provided by such systems, processes or programs) described herein.

The techniques described in this disclosure, including those attributed to the systems, or various constituent components, may be implemented, at least in part, in hardware, software, firmware, or any combination thereof. For example, various aspects of the techniques may be implemented by the computing apparatus **102,** which may use one or more processors such as, e.g., one or more microprocessors, DSPs, ASICs, FPGAs, CPLDs, microcomputing apparatus, or any other equivalent integrated or discrete logic circuitry, as well as any combinations of such components, image processing devices, or other devices. The term "processing apparatus," "processor," or "processing circuitry" may generally refer to any of the foregoing logic circuitry, alone or in combination with other logic circuitry, or any other equivalent circuitry. Additionally, the use of the word "processor" may not be limited to the use of a single processor but is intended to connote that at least one processor may be used to perform the techniques and processes described herein.

Such hardware, software, and/or firmware may be implemented within the same device or within separate devices to support the various operations and functions described in this disclosure. In addition, any of the described components may be implemented together or separately as discrete but interoperable logic devices. Depiction of different features, e.g., using block diagrams, etc., is intended to highlight different functional aspects and does not necessarily imply that such features must be realized by separate hardware or software components. Rather, functionality may be performed by separate hardware or software components or integrated within common or separate hardware or software components.

When implemented in software, the functionality ascribed to the systems, devices and techniques described in this disclosure may be embodied as instructions on a computer-readable medium such as RAM, ROM, NVRAM, EEPROM, FLASH memory, magnetic data storage media, optical data storage media, or the like. The instructions may be executed by the computing apparatus **102** to support one or more aspects of the functionality described in this disclosure.

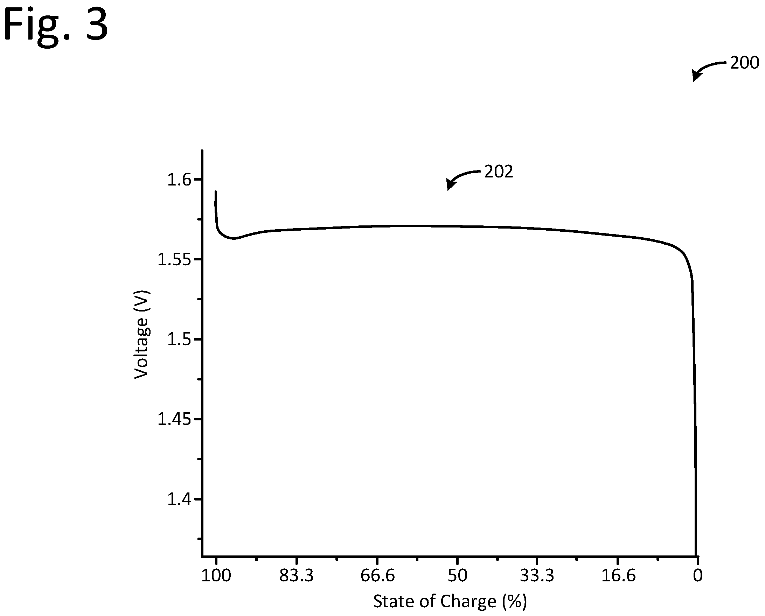

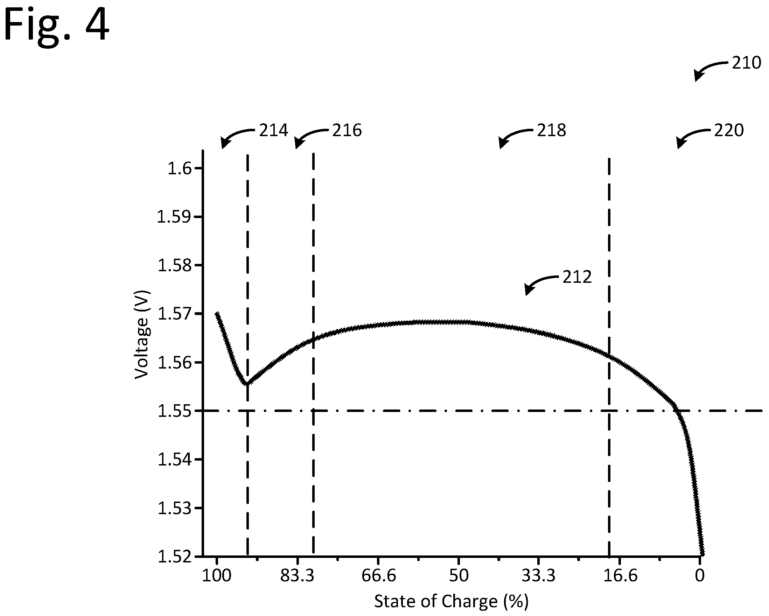

**FIG. 3** and **4** depict graphs **200, 210** of exemplary discharge voltage curves **202, 212** of zinc/silver oxide batteries (e.g., battery **116** of **FIG. 1** and **2****).** **FIG. 3** shows graph **200** of an exemplary discharge voltage curve **202** of a zinc/silver oxide battery. **FIG. 4** shows a graph **210** of another exemplary discharge voltage curve **212** of a zinc/silver oxide battery, vertically zoomed in. Each of graphs **200** and **210** depict the voltage of a zinc/silver battery in volts on the y-axis and a percent state of charge on the x-axis. The values depicted in graphs **200** and **210** are exemplary and batteries may deviate from the specific values depicted therein depending on their nominal voltages and maximum capacities.

As used herein, "discharge voltage curve" refers to the voltage of a battery (e.g., battery **116** of **FIG. 1** and **2****)** at a given state of charge of the battery. The voltage of a battery may vary at various states of charge of the battery. The battery may have a maximum voltage at 100 percent state of charge and a minimum voltage at 0 percent state of charge. However, the voltage may not be directly proportional to the state of charge. In other words, as the battery discharges the voltage does not merely diminish. Instead, the battery voltage may drop, rise, and/or remain steady as the battery is discharged.

In addition to graph **210** being vertically zoomed in relative to graph **200,** the discharge voltage curve **212** is also divided into four portions **214, 216, 218, 220.** A first portion **214** of the discharge voltage curve **212** corresponds to the state of charge of the battery being above about 93 percent (e.g., 0-4 milliamp hours discharged when the battery has a total capacity of 60 milliamp hours). A second portion **216** of the discharge voltage curve **212** corresponds to the state of charge of the battery being above about 80 percent and below about 93 percent (e.g., 4-12 milliamp hours discharged when the battery has a total capacity of 60 milliamp hours). A third portion **218** of discharge voltage curve **212** corresponds to the state of charge of the battery being above about 20 percent and below about 80 percent state of charge (e.g., 12 to 48 milliamps discharged when the battery has a total capacity of 60 milliamp hours). A fourth portion **220** of discharge voltage curve **212** corresponds to the state of charge of the battery being below 20 percent state of charge (e.g., 48-60 milliamps discharged when the battery has a total capacity of 60 milliamp hours).

**As** shown in each of the discharge voltage curves **202, 212,** the voltage of the zinc/silver batteries fluctuates between about 1.55 volts and 1.57 volts. Additionally, the voltage of each of the batteries drops quickly when beginning to discharge at full capacity (e.g., about 100 percent state of charge). After about 6.6 percent of the capacity (e.g., 4 milliamp hours when the battery has a total capacity of 60 milliamp hours) is discharged from the batteries, the voltage of the batteries begins to increase. The voltage of the batteries increases until about 20 percent of the capacity (e.g., 12 milliamp hours when the battery has a total capacity of 60 milliamp hours) is discharged from the batteries. At this point, the voltage may be relatively static (e.g., fluctuate less than 5 millivolts) until about 80 percent of the capacity (e.g., about 18 milliamp hours when the battery has a total capacity of 60 milliamp hours) is discharged from the batteries. At above about 80 percent of the capacity (e.g., about 48 milliamp hours when the battery has a total capacity of 60 milliamp hours) discharged, the voltage of the battery decreases until the battery is fully discharged.

Batteries such as battery **116** of **FIG. 1** and **2** and zinc/silver oxide batteries may be used in implantable medical devices, remote sensors, or in other devices for applications that make frequent battery charging or replacement unfavorable. In general, batteries used in such devices may be considered unsuitable for use below 80 percent state of charge. As can be seen from graphs **200** and **210,** the voltage of such batteries ranges from 1.55 volts and 1.57 volts when the batteries are between 80 percent state of charge and 100 percent state of charge, and when the batteries are between 20 percent state of charge and 80 percent state of charge. Accordingly, a single voltage measurement is insufficient to ensure that such batteries have a state of charge of at least 80 percent state of charge. Thus, a single voltage measurement may not be sufficient to determine a health of such batteries and whether such batteries are suitable for use.

However, it can be seen from graph **210** that a rate of voltage change of zinc/silver oxide differs more significantly as such batteries discharge than the voltage of the batteries. The rate of voltage change of the batteries at greater than 80 percent state of charge (portions **214, 216)** differs significantly from a rate of voltage change between 20 percent state of charge and 80 percent state of charge (the third portion **218).** As shown, the second portion **216** has a positive rate of voltage change greater than any part of the third portion **218** and any part of the fourth portion **220.** Accordingly, a determined rate of voltage change for such batteries that is above a positive rate threshold indicates that the battery has a state of charge in the second portion **216** and, therefore, is above 80 percent state of charge. Furthermore, the first portion **214** has a negative rate of voltage change less than any part of the second portion **216** or third portion **218.** However, a part of the fourth portion **220** has a negative rate of voltage change that could be equal to or less than that of the first portion **214.** Accordingly, a determined rate of voltage change for zinc/silver oxide batteries below a negative rate of change threshold may indicate that such battery has a state of charge in the first portion **214** or the fourth portion **220.**

**To** determine whether a zinc/silver battery with a rate of voltage change below the negative rate threshold has a state of charge in the first portion **214** or the fourth portion **220,** a voltage measurement of such battery may be compared to a voltage threshold. As shown in graph **210,** the fourth portion **220** has a rate of voltage change similar to the rate of voltage change of the first portion **214** when the battery voltage is below 1.55. Furthermore, no other portion of the discharge voltage curve **212** falls below 1.55 volts. Accordingly, the zinc/silver oxide batteries with a voltage below 1.55 volts can be considered to have a state of charge of less than 20 percent. Thus, using a voltage threshold of 1.55 volts, zinc/silver batteries can immediately be deemed unsuitable for use when a voltage measurement indicates such batteries have a voltage less than the voltage threshold. Accordingly, batteries with a measured voltage greater than or equal to the voltage threshold and a determined rate of voltage change less than the negative rate of change threshold can be considered to have a state of charge corresponding to the first portion **214** (e.g., a state of charge greater than 93 percent).

Although voltage values (e.g., 1.55 volts) and delivered capacities (e.g., 60 milliamp hours) used in the examples described above are specific to some zinc/oxygen batteries, the general principles can be applied to batteries with a relatively flat discharge voltage curve. For example, the voltage threshold can be adjusted based on the nominal voltage of a particular battery or battery chemistry. Furthermore, the state of charge percentages can apply batteries with varying capacities, not just those with a maximum power capacity of 60 milliamp hours.

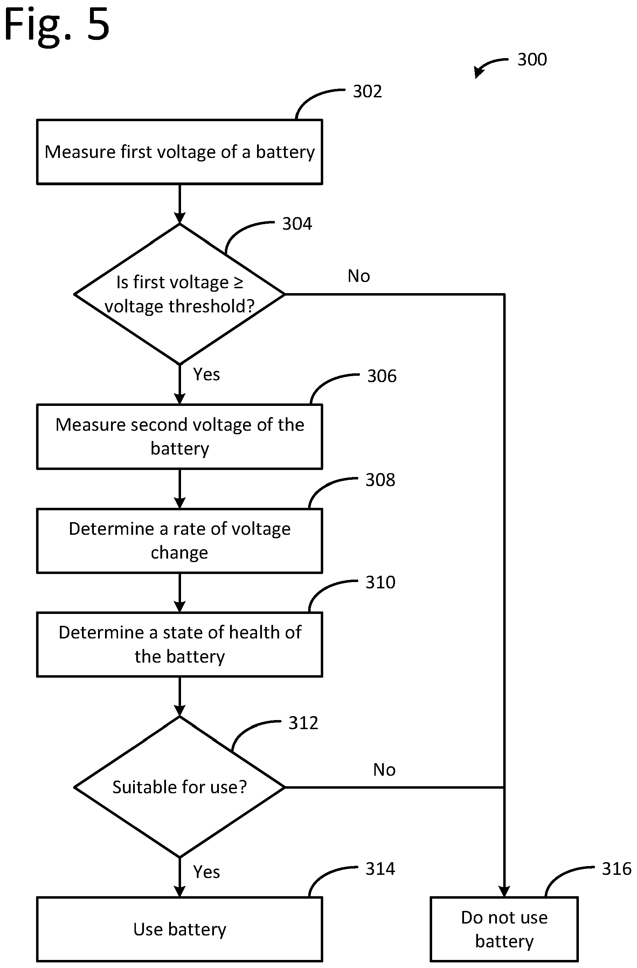

Advantageously, the health of batteries having a relatively flat discharge voltage curve such as, for example, zinc/silver oxide batteries, can be determined based on a first measured voltage and a rate of voltage discharge. A method or process for determining the health of such batteries is depicted in **FIG. 5****.**

**FIG. 5** shows a flow diagram of an embodiment of a method or process **300** for determining a state of health of batteries with a relatively flat discharge voltage curve.

At **302,** a first voltage of the battery may be measured. The first voltage may be measured using one or more of a voltage sensor (e.g., voltage sensor **110** of **FIG. 1** and **2****),** a battery measurement device or system (e.g., battery measurement system **100** of **FIG. 1** and **2****),** a multimeter, or other suitable device for measuring the voltage of a battery. The voltage may be measured by taking the difference of a voltage of the positive terminal of the battery and a voltage of the negative terminal of the battery.

At **304,** it may be determined whether the first voltage is equal to or greater than a voltage threshold. The voltage threshold may be based, at least in part, on a nominal voltage of the battery. The voltage threshold may be at least 1.55 volts and no greater than 1.60 volts (e.g., for a zinc/silver oxide battery). In one embodiment, the voltage threshold is 1.55 volts. In response to the first threshold being less than the voltage threshold, it may be determined that the state of health of the battery is poor and that such battery is not suitable for use. Accordingly, the battery may not be used in an implantable medical devices, remote sensors, or in other devices for applications that make frequent battery charging or replacement unfavorable. In response to the first threshold being greater than or equal to the voltage threshold, a second voltage of the battery may be measured at **306.**

At **306,** the second voltage of the battery may be measured at the conclusion of a predetermined time period. The predetermined time period may begin when the first voltage is measured. The predetermined time period may be any suitable time period to allow an accurate determination or estimation of a rate of voltage change. The predetermined time period may be based on a typical rate of self-discharge of the battery **116.** The predetermined time period may be based on a rate of discharge of the battery connected to a load (e.g., when incorporated into a device). The rate of discharge of the battery when connected to the load may be determined using ohms law. The predetermined time period may be at least 24 hours. The battery may self-discharge during the predetermined time period. The battery may be discharged via a connected load during the predetermined time period. The second voltage may be measured using any of the techniques described herein with regard to the first voltage.

Additional voltage measurements may be taken during the predetermined time period to provide a set of voltages. Furthermore, a set of time stamps may be recorded. Each time stamp of the set of time stamps may correspond to a different voltage of the set of voltages. After at least the first voltage and the second voltage have been measured, a rate of voltage change may be determined at **308.**

At **308,** the rate of voltage change may be determined based on the first voltage, the second voltage, and the predetermined time period. The second voltage may be subtracted from the first voltage to determine a voltage difference between the first voltage and the second voltage. The voltage difference may be divided by the predetermined time period to determine the rate of voltage change. Accordingly, the rate of voltage change may be expressed in terms of volts per unit of time. The rate of voltage change may also be expressed in terms of volts per unit of charge. In at least one embodiment, the rate of voltage change may be further based on the set of voltage measurements and their corresponding time stamps.

A discharge voltage curve of the battery during the predetermined time period may be determined using the first voltage, the second voltage, the set of voltages, the set of time stamps, and the predetermined time period. The discharge voltage curve may be determined using any suitable curve fitting algorithm or software with using the first voltage, the second voltage, the set of voltages, the set of time stamps, and the predetermined time period as inputs. The determined discharge voltage curve may be compared to a discharge voltage curve model. The rate of voltage change may be determined based on the determined discharge voltage curve. The rate of voltage change may be determined based on an average slope of the determined discharge voltage curve. After the rate of discharge is determined, a health of the battery may be determined at **310.**

At **310,** the state of health of the battery may be determined based on the rate of voltage change. The rate of voltage change may be compared to one or more rate of change thresholds. In one or more embodiments, the one or more rate of change thresholds includes a negative rate of change threshold and a positive rate of change threshold. The state of health of the battery may be determined to be poor when the rate of change is greater than or equal to the negative rate of change threshold and less than or equal to the positive rate of change threshold. Conversely, the state of health of the battery may be determined to be good when the rate of change is less than the negative rate of change threshold or greater than the positive rate of change threshold.

As described above with regards to **FIG. 3** and **4****,** a rate of voltage change less than the negative rate of change threshold or greater than the positive rate of change threshold indicates that the battery is at greater than or equal to 80 percent state of charge. The state of health of a battery at greater than or equal to 80 percent state of charge after being stored for a time period may be determined to be good. Conversely, the state of health of a battery at less than 80 percent state of charge after being stored for the time period may be determined to be good. The time period that a battery may be stored may be up to 18 months.

The negative rate of change threshold may be based on the model discharge voltage curve of the battery when such discharge voltage curve is less than the voltage threshold. In one or more embodiments, the negative rate of change threshold is -1 microvolt per hour. The positive rate of change threshold may be based on the positive rate of voltage change of a portion of the model discharge voltage curve corresponding to a state of charge of greater than 80 percent. In one or more embodiments, the positive rate of change threshold is 1 microvolt per hour. After the state of health of the battery is determined, it can be determined whether the battery is suitable for use at **312.**

At **312,** whether the battery is suitable for use can be determined based on the health of the battery. If the health of the battery is poor, it may be determined that the battery is not suitable for use in any suitable device. Accordingly, the battery may not be used at **316.** If the battery is determined to be healthy or have a state of health that is good, it may be determined that the battery is suitable for use in any suitable device. Accordingly, the battery may be used at 314. Suitable devices may include, for example, implantable medical devices, remote sensors, or other devices for applications that make frequent battery charging or replacement unfavorable. In at least one embodiment, it may be determined that the battery is suitable for use in an implantable medical device in response to the rate of voltage change being less than the negative rate of change threshold or greater than the positive rate of change threshold.

At **314,** the battery may be used in any suitable device in response to determining the battery is suitable for use. In at least one embodiment, an implantable medical device including the battery may be implanted in a patient in response to determining the battery is suitable for use in the implantable medical device. Implantable medical devices may include a pacemaker, a glucose monitor, or other implantable medical device.

At **316,** the battery is not used in response to determining the battery is unsuitable for use. The battery may be unsuitable for use if the first voltage is less than the voltage threshold. The battery may be unsuitable for use if the rate of voltage change of the battery is greater than or equal to the negative rate of change threshold or less than and equal to the positive rate of change threshold. Not using the battery may include discarding the battery, replacing the battery out of a given device, or discarding the given device when the battery is not replaceable.

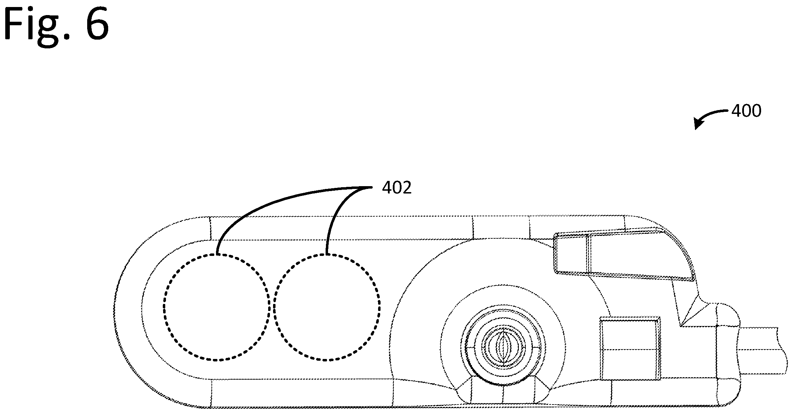

Batteries, such as battery **116 of** **FIG. 1** and **2****,** may be used in implantable medical devices. **FIG.** 6 shows an implantable medical device **400** that includes batteries 402.

The implantable medical device **400** can include any suitable medical device that is adapted to be implanted within a body of a patient. In one or more embodiments, the device **400** can be a pacemaker. Further, in one or more embodiments, the device **400** can be a leadless cardiac monitor. The device **400** can include any other suitable medical devices such as, for example, at least one of a glucose monitor, defibrillator, LVAD, neurostimulator, drug pump, diagnostic device, implantable pulse generator, etc.

Implantable pacemakers may include an electronic assembly disposed within a housing and one or more electrical leads. The electronic assembly may, for example, be implanted in a small pocket in the patient's chest. The electronic assembly may include the batteries **402.** The electrical leads can be coupled to the electronic assembly, which may include one or more electronic components that generate pacing pulses and/or sense cardiac electrical activity. The electrical leads may extend from the electronic assembly to a target site (e.g., an atrium and/or a ventricle) such that electrodes disposed on or within the electrical leads are positioned at the target site. The electronic assembly may provide electrical stimulation to the target site and/or monitor cardiac electrical activity at the target site via the electrodes.

Such implantation of the electronic assembly in a small pocket in the patient's chest may render replacement of batteries **402** unfavorable after implantation. Accordingly, it may be advantageous to determine a health of the batteries **402** prior to implantation of the implantable pacemaker (e.g., implantable medical device **400).**

The batteries **402** may include any suitable battery with a relatively flat voltage curve. In one embodiment, the batteries **402** include zinc/silver oxide batteries. A state of health of the batteries **402** may be determined. The state of health of batteries **402** may be determined using the methods and processes described herein, for example, the method 300. Additionally, the state of health of the batteries **402** may be determined using the systems and devices described herein, for example, the battery measurement system **100** of **FIG. 1** and **2****.** Whether to implant the device **400** may be determined based on the state of health of one or more of the batteries **402.** If the state of health of both of the batteries is determined to be good (e.g., a state of charge of 80 percent or more), the implantable medical device **400** may be implanted in a patient including the batteries **402.** If the state of health of one or more of the batteries is determined to be poor (e.g., a state of charge of less than 80 percent), the device **400** may be discarded with the batteries **402.** Alternatively, each of the unhealthy batteries of the batteries **402** may be replaced with healthy batteries.

The invention is defined in the claims.

All scientific and technical terms used herein have meanings commonly used in the art unless otherwise specified. The definitions provided herein are to facilitate understanding of certain terms used frequently herein and are not meant to limit the scope of the present disclosure.

As used herein, singular forms "a," "an" and "the" include plural referents unless the context clearly dictates otherwise. As used in this specification and the appended claims, the term "or" is generally employed in its sense including "and/or" unless the content clearly dictates otherwise. The term "and/or" means one or all of the listed elements or a combination of any two or more of the listed elements.

The words "preferred" and "preferably" refer to embodiments of the disclosure that may afford certain benefits, under certain circumstances. However, other embodiments may also be preferred, under the same or other circumstances. Furthermore, the recitation of one or more preferred embodiments does not imply that other embodiments are not useful and is not intended to exclude other embodiments from the scope of the inventive technology.

Unless otherwise expressly stated, it is in no way intended that any method set forth herein be construed as requiring that its steps be performed in a specific order. Accordingly, where a method claim does not actually recite an order to be followed by its steps or it is not otherwise specifically stated in the claims or descriptions that the steps are to be limited to a specific order, it is no way intended that any particular order be inferred. Any recited single or multiple feature or aspect in any one claim can be combined or permuted with any other recited feature or aspect in any other claim or claims.

It will be apparent to those skilled in the art that various modifications and variations can be made to the present inventive technology without departing from the scope of the disclosure. Since modifications, combinations, subcombinations and variations of the disclosed embodiments incorporating the substance of the inventive technology may occur to persons skilled in the art, the inventive technology should be construed to include everything within the scope of the appended claims.

## Claims

1. A battery measurement system comprising:
a positive node (112) configured to operatively couple to a positive terminal of a battery (116) and provide a first voltage signal indicative of a positive terminal voltage of the battery;
a negative node (114) configured to operatively couple to a negative terminal of the battery (116) and provide a second voltage signal indicative of a negative terminal voltage of the battery;
a voltage sensor (110) operatively coupled to the positive node and the negative node to receive the first and second voltage signals and configured to:
determine a voltage of the battery based on the first and second voltage signals; and
provide a battery voltage signal; and
a computing apparatus (102) comprising one or more processors operatively coupled to the voltage sensor and configured to:
measure a first voltage of the battery;
determine whether the first voltage is equal to or greater than a voltage threshold level;
measure a second voltage of the battery at a conclusion of a predetermined time period in response to the first voltage being equal to or greater than the voltage threshold level, the predetermined time period beginning when the first voltage is measured;
determine a rate of voltage change based on the first voltage, the second voltage, and the predetermined time period; and
determine a state of health of the battery based on the rate of voltage change.

2. The system of claim 1, wherein to determine the state of health of the battery the computing apparatus is configured to compare the rate of voltage change to a rate of voltage change model.

3. The system of claim 1, wherein to determine the state of health of the battery the computing apparatus is configured to compare the rate of voltage change to one or more rate of change thresholds.

4. The system of claim 3, wherein the one or more rate of change thresholds comprises:
a negative rate of change threshold; and
a positive rate of change threshold.

5. The system of claim 4, wherein the computing apparatus is further configured to determine whether the battery is suitable for use in an implantable medical device (400) in response to the rate of voltage change being less than the negative rate of change threshold or greater than the positive rate of change threshold.

6. The system of any one of claims 1-3, wherein the computing apparatus is further configured to determine whether the battery is suitable for use in an implantable medical device (400) based on the state of health of the battery.

7. The system of any one of claims 1-3, wherein the predetermined time period is at least 24 hours.

8. The system of any one of claims 1-3, wherein the computing apparatus is configured to allow the battery to self-discharge during the predetermined time period.

9. The system of any one of claims 1-3, further comprising the battery and the battery comprises a zinc/silver oxide battery.

10. A pacemaker (400) comprising the battery measurement system of any of one of claims 1-9.

## Patentansprüche

1. Batteriemesssystem, Folgendes umfassend:
einen positiven Knoten (112), der dazu ausgelegt ist, operativ mit einem positiven Anschluss einer Batterie (116) zu koppeln und ein erstes Spannungssignal bereitzustellen, das eine positive Anschlussspannung der Batterie angibt;
einen negativen Knoten (114), der dazu ausgelegt ist, operativ mit einem negativen Anschluss der Batterie (116) zu koppeln und ein zweites Spannungssignal bereitzustellen, das eine negative Anschlussspannung der Batterie angibt;
einen Spannungssensor (110), der operativ mit dem positiven Knoten und dem negativen Knoten gekoppelt ist, um das erste und das zweite Spannungssignal zu empfangen, und ausgelegt ist, um:
eine Spannung der Batterie basierend auf dem ersten und dem zweiten Spannungssignal zu bestimmen; und
ein Batteriespannungssignal bereitzustellen; und
eine Rechenvorrichtung (102), die einen oder mehrere Prozessoren umfasst, die operativ mit dem Spannungssensor gekoppelt sind und ausgelegt sind, um:
eine erste Spannung der Batterie zu messen;
zu bestimmen, ob die erste Spannung gleich oder größer als ein Spannungsschwellenpegel ist;
eine zweite Spannung der Batterie am Ende einer vorbestimmten Zeitdauer als Reaktion darauf zu messen, dass die erste Spannung gleich oder größer als der Spannungsschwellenpegel ist, wobei die vorbestimmte Zeitdauer beginnt, wenn die erste Spannung gemessen wird;
eine Spannungsänderungsrate basierend auf der ersten Spannung, der zweiten Spannung und der vorbestimmten Zeitdauer zu bestimmen; und
einen Gesundheitszustand der Batterie basierend auf der Spannungsänderungsrate zu bestimmen.

2. System nach Anspruch 1, wobei zum Bestimmen des Gesundheitszustands der Batterie die Rechenvorrichtung dazu ausgelegt ist, die Spannungsänderungsrate mit einem Spannungsänderungsratenmodell zu vergleichen.

3. System nach Anspruch 1, wobei zum Bestimmen des Gesundheitszustands der Batterie die Rechenvorrichtung dazu ausgelegt ist, die Spannungsänderungsrate mit einem oder mehreren Änderungsratenschwellenwerten zu vergleichen.

4. System nach Anspruch 3, wobei die eine oder die mehreren Änderungsratenschwellenwerte Folgendes umfassen:
einen negativen Änderungsratenschwellenwert; und
einen positiven Änderungsratenschwellenwert.

5. System nach Anspruch 4, wobei die Rechenvorrichtung ferner dazu ausgelegt ist, als Reaktion darauf, dass die Spannungsänderungsrate kleiner ist als der negative Änderungsratenschwellenwert oder größer ist als der positive Änderungsratenschwellenwert, zu bestimmen, ob die Batterie zur Verwendung in einer implantierbaren medizinischen Vorrichtung (400) geeignet ist.

6. System nach einem der Ansprüche 1 bis 3, wobei die Rechenvorrichtung ferner dazu ausgelegt ist, basierend auf dem Gesundheitszustand der Batterie zu bestimmen, ob die Batterie zur Verwendung in einer implantierbaren medizinischen Vorrichtung (400) geeignet ist.

7. System nach einem der Ansprüche 1 bis 3, wobei der vorbestimmte Zeitraum mindestens 24 Stunden beträgt.

8. System nach einem der Ansprüche 1 bis 3, wobei die Rechenvorrichtung dazu ausgelegt ist, der Batterie zu ermöglichen, sich während des vorbestimmten Zeitraums selbst zu entladen.

9. System nach einem der Ansprüche 1 bis 3, ferner die Batterie umfassend, wobei die Batterie eine Zink/Silberoxidbatterie umfasst.

10. Schrittmacher (400), das Batteriemesssystem nach einem der Ansprüche 1 bis 9 umfassend.

## Revendications

1. Système de mesure de batterie comprenant :
un nœud positif (112) configuré pour se coupler fonctionnellement à une borne positive d'une batterie (116) et fournir un premier signal de tension indicatif d'une tension de borne positive de la batterie ;
un nœud négatif (114) configuré pour se coupler fonctionnellement à une borne négative de la batterie (116) et fournir un second signal de tension indicatif d'une tension de borne négative de la batterie ;
un capteur de tension (110) couplé de manière fonctionnelle au nœud positif et au nœud négatif pour recevoir les premier et second signaux de tension et configuré pour :
déterminer une tension de la batterie sur la base des premier et second signaux de tension ; et
fournir un signal de tension de batterie ; et
un appareil de calcul (102) comprenant un ou plusieurs processeurs couplés de manière fonctionnelle au capteur de tension et configurés pour :
mesurer une première tension de la batterie ;
déterminer si la première tension est égale ou supérieure à un niveau seuil de tension ;
mesurer une seconde tension de la batterie à l'issue d'un intervalle de temps prédéterminé en réponse à la première tension qui est égale ou supérieure au niveau seuil de tension, l'intervalle de temps prédéterminé débutant lorsque la première tension est mesurée ;
déterminer un taux de variation de tension sur la base de la première tension, de la seconde tension et de la période de temps prédéterminée ; et
déterminer un état de santé de la batterie sur la base du taux de variation de tension.

2. Système selon la revendication 1, pour déterminer l'état de santé de la batterie, l'appareil de calcul étant configuré pour comparer le taux de variation de tension à un modèle de taux de variation de tension.

3. Système selon la revendication 1, pour déterminer l'état de santé de la batterie, l'appareil de calcul étant configuré pour comparer le taux de variation de tension à un ou plusieurs seuils de taux de variation.

4. Système selon la revendication 3, le ou les seuils de taux de variation comprenant :
un seuil de taux de variation négatif ; et
un seuil de taux de variation positif.

5. Système selon la revendication 4, l'appareil de calcul étant en outre configuré pour déterminer si la batterie est appropriée pour une utilisation dans un dispositif médical implantable (400) en réponse au fait que le taux de variation de tension est inférieur au seuil de taux de variation négatif ou supérieur au seuil de taux de variation positif.

6. Système selon l'une quelconque des revendications 1 à 3, l'appareil de calcul étant en outre configuré pour déterminer si la batterie est appropriée pour une utilisation dans un dispositif médical implantable (400) sur la base de l'état de santé de la batterie.

7. Système selon l'une quelconque des revendications 1 à 3, la période de temps prédéterminée étant d'au moins 24 heures.

8. Système selon l'une quelconque des revendications 1 à 3, l'appareil de calcul étant configuré pour permettre à la batterie de se décharger automatiquement pendant la période de temps prédéterminée.

9. Système selon l'une quelconque des revendications 1 à 3, comprenant en outre la batterie et la batterie comprenant une batterie zinc/oxyde d'argent.

10. Stimulateur cardiaque (400) comprenant le système de mesure de batterie selon l'une quelconque des revendications 1 à 9.
